Europäisches Patentamt

European Patent Office

Office européen des brevets

(19)

(11) Publication number : **0 380 880 B1**

(12) **EUROPEAN PATENT SPECIFICATION**

(45) Date of publication of patent specification :
**31.03.93 Bulletin 93/13**

(51) Int. Cl.$^5$ : **A61K 31/35,** A61K 9/12,
A61K 9/72

(21) Application number : **89313152.4**

(22) Date of filing : **15.12.89**

(54) **Aerosol compositions containing sodium chromoglycate.**

The file contains technical information
submitted after the application was filed and
not included in this specification

(30) Priority : **16.12.88 GB 8829478**

(43) Date of publication of application :
**08.08.90 Bulletin 90/32**

(45) Publication of the grant of the patent :
**31.03.93 Bulletin 93/13**

(84) Designated Contracting States :
**AT BE CH DE FR GB IT LI LU NL SE**

(56) References cited :
**FR-A- 2 339 604**

(73) Proprietor : **NORTON HEALTHCARE LIMITED
Gemini House Flex Meadow
Harlow Essex CM19 5TJ (GB)**

(72) Inventor : **Bell, John Howard
34 Fairmount Drive
Loughborough Leicestershire LE11 3JR (GB)**

(74) Representative : **Green, Mark Charles et al
Urquhart-Dykes & Lord, 91 Wimpole Street
London W1M 8AH (GB)**

EP 0 380 880 B1

## Description

The present invention relates to formulations more particularly, aerosol formulations. In particular it relates to aerosol formulations containing sodium cromoglycate.

Sodium cromoglycate has been used in aerosol formulations for some time. According to the literature, there are many problems in formulating sodium cromoglycate particularly in the aerosol form. Such problems include the particle size of the pharmaceutical powder which is incorporated in medicaments used for inhalation therapy.

The technology of the preparation of pharmaceutical inhalation aerosols in pressurised packs is comprehensively described in GB 837465. Attention is drawn to the need to use micronised drug particles in a size range less than 25 microns and the requirement to control the moisture content of the formulation to low levels, 300 parts per million by weight of the total composition being specifically mentioned.

GB 1242211 indicates that medicaments for administration by inhalation should be of a controlled particle size in order to achieve maximum penetration into the lungs; a suitable particle size range being 0.01 to 10 microns, usually 1 to 10 µm. Fluidization is said to be a problem in powder inhalers and the problem is overcome by the addition of a pharmaceutical carrier having an effective particle size in the range 30 to 80 µm.

GB 1562901 also makes it clear that a large proportion of water in sodium cromoglycate is disadvantageous to the composition. In particular according to GB 1562901, it is important that a pharmaceutical composition comprising from 1 to 20% by weight of finely divided sodium cromoglycate and liquefied propellants, the composition showed contains less than 4% by weight of water.

Three chlorofluorocarbon propellants have become commonly usd in medicinal pressurised aerosols, trichloromonofluoromethane [propellant 11, Freon 11,Arcton 11], dichlorodifluoromethane [propellant 12, Freon 12, Arcton 12] and dichlorotetrafluoroethane [propellant 114, Freon 114, Arcton 114]. Propellant 11 is of particular interest because it is liquid at room temperatures, which allows especially cost effective manufacturing techniques to be applied. Propellant 12 and 114 are gaseous at ambient temperatures and require special handling which adds to production difficulties.

US 4405598 and GB 2001334B make it clear that there are problems with certain propellants, in particular propellant 11 [trichloromonofluoromethane]. Although US 4405598 states that propellant 11 has undesirable environmental effects, the propellant is no different than other halogenated hydrocarbons as far as damage to the environment is concerned. In fact, propellant 11 is very extensively used with medicaments such as salbutamol. There are no significant toxic side effects which hinder its very successful clinical use.

However according to Remington's Pharmaceutical Sciences Chapter 92, page 1655, propellant 11 is particularly sensitive to hydrolysis, leading to the production of hydrochloric acid which would be detrimental to the product itself and would preclude use by patients in medical products. U.K. 1429184 describes the growth of drugs suspended in propellant 11 due to the formulation of a solvate between the two substances; such growth is detrimental to inhalation aerosols.

The drug substance sodium cromoglycate is an important therapeutic agent in asthma. According to US 4405598 its formulation into an aerosol is especially difficult and this is supported by papers in the scientific literature such as J. PHARM PHARMAC 1973, 25 96P - 103P and J.PHARM.SCI. 1971 60 1459-1465, where unusual moisture properties of the drug are described. The drug substance is capable of absorbing considerable quantities of moisture into its crystal lattice.

This unusual property of sodium cromoglycate makes the preparation of pressurised aerosols unusually difficult in the light of the teachings of US 4405598. GB 2001334B reveals that the use of propellants 12 and 114 are necessary, and propellant 11 must be specifically excluded. US 4405598 further teaches that a carefully controlled moisture content of the sodium cromoglycate is required and that the presence of water at higher levels than claimed would have significant and deleterious effects on the quality of the aerosol generated by the final product.

With the above prior art in mind, the problem that the present applicants had was to formulate an effective formulation containing sodium cromoglycate in order to provide the necessary particle size in an inhalation aerosol suitable to give beneficial effects to the user from the medicament.

According to the present invention there is provided a pharmaceutical composition containing 0.1 to 10% dry sodium cromoglycate, trichloromonofluromethane and/or dichlorotetrafluoroethane in an amount of 10% to 39% by weight, dichlorodifluoromethane in an amount of 50% to 80% by weight, a surfactant in an amount of 0.1 to 1.5% by weight, all based on the weight of the total composition, and water in an amount of 2 to 14% by weight based on the weight of the dry sodium cromoglycate .

Surprisingly, we have now found that satisfactory pressurised aerosols of sodium cromoglycate can be manufactured with the use of conventional pressurised pack manufacturing technology including the use of propellant 11. Previous teaching suggests that such formulations might only be prepared using sodium cro-

EP 0 380 880 B1

moglycate of very low water content, which would for example reduce the production of acid from the hydrolysis of propellant 11, maintain proper valve action, and assist adequate suspension properties of the finely micronised drug. However, satisfactory formulations can be prepared using sodium cromoglycate with water content of from 4% to 14% by weight based on the weight of the dry sodium cromoglycate. The sodium cromoglycate content of the composition with the sodium cromoglycate being in a dry form should be in the range of 0.1% to 10% by weight of the total composition, preferably 1 to 5% by weight.

Such formulations are unusual in pressurised packs for a number of reasons. It would be expected that the valve mechanism would not function effectively due to lubrication problems. Our studies on the proposed new formulations have shown no problems on valve mechanisms provided conventional additives are used in the formulation. Such materials include sorbitan trioleatae, oleic acid, and lecithin. Repeated actuation of the valve shows no evidence of sticking or of leaking propellant gas during or after use. The valve delivers an accurate and consistent weight of suspension. Our formulations show technological advance over previous formulations in that they are cheaper to produce and do not need the elaborate precautions previously thought necessary in this field to overcome problems outlined in the prior art.

The suspensions produced from the new formulations re-suspend easily when gently agitated to give uniform milky liquids, with no evidence of claying or agglomeration of particles and there is a complete lack of any evidence of crystal growth as found and described in UK 14299184. The date of Table I illustrates the weight of product discharged from the metering valve throughout the life of the cannister even with sodium cromoglycate moisture content ranging from 1-10% w/w, there is no deleterious effect on the uniformity of the quantity of product delivered for either a 1mg or a 5mg preparation of sodium cromoglycate. This demonstrates that a uniform product is produced which delivers a constant and predictable dose throught the life of the canister.

## TABLE I

Table I shows uniformity in mean weight (mg) of the metered dose from inhalers prepared using sodium cromoglycate with 1%, 4% and 10% water content at two dose levels.

**1mg Product** (Nominal Delivery weight 68.12mg)

| Dose No. from 200 dose cannister | Water Content of Sodium Cromoglycate w/w | | |
|---|---|---|---|
| | 1% | 4% | 10% |
| 11 - 20 | 70.1 | 64.7 | 70.6 |
| 91 - 100 | 69.9 | 64.6 | 69.2 |
| 191 - 200 | 70.7 | 68.7 | 69.6 |

**5mg Product** (Nominal Deliver Weight 136.24mg)

| Dose No. from 112 dose cannister | Water Content of Sodium Cromoglycate w/w | | |
|---|---|---|---|
| | 1% | 4% | 10% |
| 11 - 15 | 139 | 136 | 139 |
| 51 - 55 | 138 | 137 | 139 |
| 90 - 94 | 136 | 140 | 140 |

The preferred surfactants include various esters such as sorbitan esters including sorbitan trioleate, sorbitan sequioleate, sorbitan mono-oleate, sorbitan monolaurate, as well as other surfactants such as oleic acid, lecithin, cetyl pyridinium chloride and sodium dioctyl sulphosuccinate.

The propellants, sodium cromoglycate and the surfactant are preferably mixed together in specific proportions to provide the desired pressure within the can in order to propel the correct particle size of sodium cromoglycate from the can for inhalation into the lungs by the user. The preferred ratio of trichloromonofluoromethane [propellant 11] to dichlorodifluoromethane [propellant 12] is in the ratio of 1;4 and calculated on a weight by weight ratio basis. Such a ratio preferably provides an internal can pressure of between 3 and 5 bar. A desirable pressure within the can would be about 4 bar or between 3.5 and 4.5 bar according to the ratio of propellant 11 to propellant 12.

In addition to propellant 12, propellant 114 may also be used. Propellant 114 is 1,2 dichloro-1,1,2, 2-tetrofluoroethane.

With regard to the particle size of sodium cromoglycate, in order that the drug may be effective when it reaches the lungs of the user, it is preferred that at least 50% of the particles of the bulk microfined sodium cromoglycate on a weight by weight basis, have a particle size of between 2-6 $\mu$m with preferably 90% of the particles on a weight by weight basis having a particle size of below 10 $\mu$m. This is achieved by grinding the drug prior to incorporation in the composition.

The mass-median diameter of the particles at this stage determined by means of a Coulter Counter is preferably in the range of 1 to 3 μm.

With regard to water content of the present invention, this generally should be in the range of 4 to 10% based on the weight of the dry sodium cromoglycate. The requirement for a controlled water content of powders in pressurised aerosols is well known. For example Remington Pharmaceuticals Sciences 13th Edition published 1965, page 661 makes it clear that the moisture content of compositions with powders contained within an aerosol should have a moisture content of below 300 ppm see [US 3014844]. It is also known that sodium cromoglycate requires a low water content- see US 4405598.

The particle sizes of the drug in the aerosol produced from a medicinal inhaler is an essential property, and it is important to ensure that varying the water content of the drug, and hence the composition, has no effect on the particle size of the drug aerosol. Inhalers were prepared using sodium cromoglycate with 1%, 4% and 10% water content and the fine particle content of the emittd aerosol measured using the two-stage impinger described for this purpose in the British Pharmacopeia 1988, Volume 2, page A204. Table II shows the weight in milligrammes of sodium cromoglycate reaching the second stage of this device.

## TABLE II

Table II shows the uniformity in mean weight (mg) of fine particles reaching stage 2 of a two-stage impinger from inhalers prepared using sodium cromoglycate with 1%, 4%, and 10% water content at two dosage levels.

### 1mg Product

| Dose No. from 200 dose canister | Water Content of Sodium Cromoglycate w/w | | |
|---|---|---|---|
| | 1% | 4% | 10% |
| 11 - 20 | 0.18 | 0.20 | 0.21 |
| 91 - 100 | 0.22 | 0.22 | 0.21 |
| 191 - 200 | 0.20 | 0.21 | 0.29 |

### 5mg Product

| Dose No. from 112 dose canister | Water Content of Sodium Cromoglycate w/w | | |
|---|---|---|---|
| | 1% | 4% | 10% |
| 11 - 15 | 0.68 | 0.58 | 0.62 |
| 51 - 55 | 0.68 | 0.66 | 0.61 |
| 90 - 94 | 0.53 | 0.62 | 0.68 |

According to Hallworth and Westmoreland [J.PHARM.PHARMAC. 1987 39 pages 966-972] this represents aerosol particles below about 6-7 μm in size which are of particular importance in medicinal aerosols. When a "1mg" product is prepared, the quantity of fine particles delivered is seen to be very constant through the life of the cannister, and over the range of water contents described. The "5mg" product shows similar consistency of dosage over the same water content range.

Surprisingly, in spite of all that has been written in relation to water content of sodium cromoglycate, we have found that using a water content of 4 to 10% by weight based on the weight of the dry sodium cromoglycate provides a suitable formulation sodium cromoglycate within the particular propellent and surfactants as described in this specification.

Examples of the present invention will now be given. All quantities are on a weight by weight basis unless otherwise stated.

## EXAMPLES 1 - 19

### Method of preparation

The surface active agent is dissolved in the propellant 11 and maintained in an insulated vessel at 5-10°C, and the sodium cromoglycate is added. This is dispersed with a high sheer mixer to produce a uniform suspension. Aliquots of the suspension are accurately measured into 18ml aluminium can, a valve is applied and crimped on. The calculated weight of propellant 12 is filled under pressure through the valve. Similary when a mixture of propellant 12 and 114 is used, the can is filled under pressure through the valve.

The surface active agent used is sorbitan trioleate, cetyl pyridinium chloride, sodium dioctyl sulphosuccinate or oleic acid.

The following table sets out the amount of each component by weight in compositions of the present invention. The water content for each example varied between 4 and 10% by weight of the sodium cromoglycate [SCG]. Each composition produced a canister dose when used.

## Examples 1 - 19

| No. | Micronised SCG | Sorbitan Trioleate | Cetyl Pyridinium Chloride | Sodium Dioctyl Sulpho Succinate | Oleic Acid | Propellant 11 | Propellant 12 | Propellant 114 |
|---|---|---|---|---|---|---|---|---|
| 1 | 1.46% | 1.02 | | | | 19.50 | 78.01 | |
| 2 | 3.67 | 1.27 | | | | 19.01 | 76.05 | |
| 3 | 2.94 | | | | 0.2 | 29.06 | 67.80 | |
| 4 | 5.00 | | 0.25 | | | 18.78 | 75.09 | |
| 5 | 1.47 | | | 0.1 | | 29.53 | 68.90 | |
| 6 | 11.76 | 5.00 | | | | 16.05 | 66.59 | |
| 7 | 3.67 | | | | 0.3 | 19.21 | 76.82 | |
| 8 | 1.47 | | 0.05 | | | 39.39 | 59.09 | |
| 9 | 2.94 | 1.50 | | | | 19.11 | 76.45 | |
| 10 | 3.67 | | | | 0.3 | 38.41 | 57.62 | |
| 11 | 1.47 | | | 0.1 | | 29.53 | 68.90 | |
| 12 | 1.47 | 1.02 | | | | 10.21 | 74.14 | 13.10 |
| 13 | 1.47 | | 0.1 | | | 25.11 | 49.18 | 24.14 |
| 14 | 2.94 | | | | 0.2 | 24.45 | 48.56 | 24.45 |
| 15 | 2.94 | 1.50 | | | | 10.21 | 62.19 | 23.16 |
| 16 | 3.67 | | | | 0.3 | 10.00 | 70.01 | 16.02 |
| 17 | 3.67 | | 0.2 | | | 23.92 | 48.12 | 24.09 |
| 18 | 14.7 | 1.02 | | | | | 48.51 | 35.77 |
| 19 | 3.67 | 2.55 | | | | | 56.27 | 37.51 |

EP 0 380 880 B1

## EXAMPLE 20

| Method of Preparation | 1mg Product | | 5mg Product | |
|---|---|---|---|---|
| Sodium cromoglycate | 1.47% | w/w | 4.04% | w/w |
| Sorbitan Tri-oleate | 1.02% | " | 1.40 | " |
| Trichlorofluoromethane | 19.50% | " | 18.91 | " |
| Dichlorodifluoromethane | 78.01% | " | 75.65 | " |

The sodium trioleate is dissolved in the trichlorofluoromethane at 8-10°C. The sodium cromoglycate is added and the suspension dispersed with a high shear mixer to produce a uniform product. Aliquots are accurately measured into aluminium canisters, and a suitable metering valve is crimped on. The calculated weight of dichlorodifluoromethane is filled under pressure through the valve. The product is stored for four weeks prior to testing.

## EXAMPLE 21

Water content of the sodium cromoglycate

The water content of the sodium cromoglycate used in the preparation is determined by loss on drying over phosphorus pentoxide as described in the British Pharmacopeia 1988, Addendum 1989 page 1173, or by Karl Fischer reagent. The water content is then adjusted downwards by controlled drying in a vacuum oven, or upwards by exposure to suitably moist air.

## EXAMPLE 22

Preparation of 1mg Inhalers with 10% water content

| Sodium cromoglycate[+] | 1.47% | w/w |
|---|---|---|
| Sorbitan Tri-oleate | 1.02% | " |
| Trichlorofluoromethane | 19.50% | " |
| Dichlorodifluoromethane | 78.01% | " |

[+] sodium cromoglycate previously adjusted to 10% w/w water content.

The method of preparation is as described in Example 20.

## Claims

1. A pharmaceutical composition containing 0.1 to 10% sodium cromoglycate on a dry basis, trichloromonofluromethane and/or dichlorotetrafluoroethane in a total amount of 10% to 39% by weight, dichlorodifluoromethane in an amount of 50% to 80% by weight, a surfactant in an amount of 0.1 to 1.5% by weight, all based on the weight of the total composition, and water in an amount of 2 to 14% by weight based on the weight of the sodium cromoglycate on a dry basis.

2. A composition as claimed in Claim 1 wherein the propellants are trichloromonofluoromethane and dichlorodifluoromethane.

3. A composition as claimed in Claim 1 wherein the propellants are trichloromonofluoromethane, dichlorodifluoromethane and dichlorotetrafluoroethane.

4. A composition as claimed in any one of the preceding claims containing 1 to 5% dry sodium cromoglycate.

5. A composition as claimed in any one of the preceding claims wherein the weight of water is 4 to 10% based on the weight of the dry sodium cromoglycate.

6. A composition as claimed in any one of the preceding claims further including sorbitan trioleate, oleic acid or lecithin as surfactant, or a mixture thereof.

**Patentansprüche**

1. Pharmazeutische Mischung, die 0.1 bis 10 % Natriumcromoglycat auf einer trockenen Basis, Trichlormonofluormethan und/oder Dichlortetrafluorethan in einer Gesamtmenge von 10 bis 39 Gew.-%, Dichlordifluormethan in einer Menge von 50 bis 80 Gew.-%, ein Tensid in einer Menge von 0.1 bis 1.5 Gew.-%, alle bezogen auf das Gewicht der gesamten Mischung, und Wasser in einer Menge von 2 bis 14 Gew.-% enthält, bezogen auf das Gewicht des Natriumcromoglycats auf einer trockenen Basis.

2. Mischung nach Anspruch 1, bei welcher die Treibmittel Trichlormonofluormethan und Dichlordifluormethan sind.

3. Mischung nach Anspruch 1, bei welcher die Treibmittel Trichlormonofluormethan, Dichlordifluormethan und Dichlordifluormethan und Dichlortetrafluorethan sind.

4. Mischung nach einem der vorhergehenden Ansprüche, welche 1 bis 5 % trockenes Natriumcromoglycat enthält.

5. Mischung nach einem der vorhergehenden Ansprüche, bei welcher das Wassergewicht 4 bis 10 % beträgt, bezogen auf das Gewicht des trockenen Natriumcromoglycats.

6. Mischung nach einem der vorhergehenden Ansprüche, welche weiterhin Sorbitontrioleat, Ölsäure oder Lecithin als ein Tensid oder ein Gemisch davon enthält.

**Revendications**

1. Composition pharmaceutique contenant de 0,1 à 10 % de cromoglycate de sodium en matière sèche, du trichloromonofluorométhane et/ou du dichlorotétrafluoroéthane en une quantité totale de 10 à 39 % en poids, du dichlorodifluorométhane en une quantité de 50 à 80 % en poids, un tensioactif en une quantité de 0,1 à 1,5 % en poids, tous par rapport au poids de la composition totale, et de l'eau en une quantité de 2 à 14 % en poids, par rapport au poids du cromoglycate de sodium en matière sèche.

2. Composition selon la revendication 1, dans laquelle les propulseurs sont le trichloromonofluorométhane et le dichlorodifluorométhane.

3. Composition selon la revendication 1, dans laquelle les propulseurs sont le trichloromonofluorométhane, le dichlorodifluorométhane et le dichlorotétrafluoroéthane.

4. Composition selon l'une quelconque des revendications précédentes, contenant de 1 à 5 % de cromoglycate de sodium sec.

5. Composition selon l'une quelconque des revendications précédentes, dans laquelle le poids d'eau est de 4 à 10 %, par rapport au poids du cromoglycate de sodium sec.

6. Composition selon l'une quelconque des revendications précédentes, comprenant du trioléate de sorbitanne, de l'acide oléique ou de la lécithine en tant que tensioactif, ou un mélange de ceux-ci.